Europäisches Patentamt
European Patent Office
Office européen des brevets

(19)

(11) Veröffentlichungsnummer: **0 434 175 A1**

(12) **EUROPÄISCHE PATENTANMELDUNG**

(21) Anmeldenummer: **90250322.6**

(51) Int. Cl.[5]: **C07D 207/08**

(22) Anmeldetag: **20.12.90**

(30) Priorität: **21.12.89 DD 336089**

(43) Veröffentlichungstag der Anmeldung: **26.06.91 Patentblatt 91/26**

(84) Benannte Vertragsstaaten: **BE CH DE ES FR GB IT LI NL**

(71) Anmelder: **INSTITUT FÜR CHEMISCHE TECHNOLOGIE**
**Rudower Chaussee 5**
**O-1199 Berlin(DE)**

Anmelder: **ZENTRALINSTITUT FÜR ORGANISCHE CHEMIE**
**Rudower Chaussee 5**
**O-1199 Berlin(DE)**

(72) Erfinder: **Ballschuh, Detlef, Dr.**
**Graudenzer Strasse 17**
**O-1034 Berlin(DE)**
Erfinder: **Ohme, Roland, Dr.**
**Waldstrasse 6**
**O-1180 Berlin(DE)**
Erfinder: **Seibt, Horst, Dr.**
**Eugen-Schönhaar-Strasse 15**
**O-1055 Berlin(DE)**
Erfinder: **Gründemann, Egon, Dr.**
**Waldstrasse 4**
**O-1180 Berlin(DE)**

(54) **Sulfobetainsubstituierte alpha-Sulfonylcarbonsäuren aus Diallylammoniumsalzen und Verfahren zu ihrer Herstellung.**

(57) Die Erfindung betrifft neue sulfobetainsubstituierte α-Sulfonylcarbonsäuren der allgemeinen Formel I und Verfahren zu ihrer Herstellung. Die Verbindungen nach Formel I stellen als organische Zwischenprodukte einen reaktiven Synthesebaustein mit Sulfobetaincharakter dar und können für weiterführende Synthesen eingesetzt werden. Bei langkettigem Alkylrest $R_1$ werden polyfunktionelle Tenside erhalten. Erfindungsgemäß werden molare Mengen von Diallylammoniumchlorid mit Chloressigsäure und der doppeltmolaren Menge Natriumhydrogensulfit in Gegenwart einer katalytischen Menge eines Peroxodisulfats miteinander umgesetzt und die erhaltene Reaktionslösung nach Zugabe einer katalytischen Jodidmenge durch Erwärmen zu sulfobetainsubstituierten α-Sulfonylessigsäuren gemäß der allgemeinen Formel I umgewandelt.

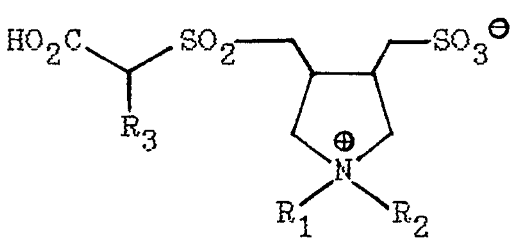

I

EP 0 434 175 A1

# SULFOBETAINSUBSTITUIERTE ∝-SULFONYLCARBONSÄUREN AUS DIALLYLAMMONIUMSALZEN UND VERFAHREN ZU IHRER HERSTELLUNG

Die Erfindung betrifft neue sulfobetainsubstituierte ∝-Sulfonylcarbonsäuren der allgemeinen Formel I,

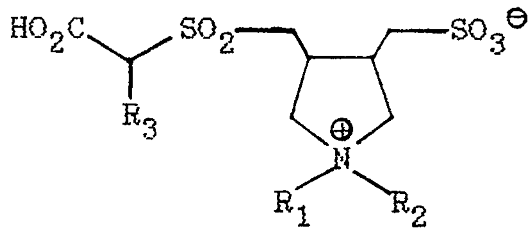

in welcher
$R_1$ und $R_2$ Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellen, oder einen -(2'/3'-Carboxymethyl-sulfonylmethyl-3'/2'-sulfomethyl)tetramethylen-Rest bedeuten,
und
$R_3$ Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellt.

Die ∝-Sulfonylcarbonsäuren der Formel I stellen als organische Zwischenprodukte mit Sulfobetaincharakter einen reaktiven Baustein für weiterführende Synthesen dar. Ist mindestens einer der Substituenten $R_1$ bis $R_3$ ein langkettiger Alkylrest, so können diese Verbindungen als polyfunktionelle Tenside (anionische Sulfobetainsulfone) Verwendung finden.

Sulfobetainsubstituierte ∝-Sulfonylcarbonsäuren der Formel I sowie Verfahren zu ihrer Herstellung sind bisher nicht bekannt geworden. Andere Alkyl- bzw. Aryl-∝-sulfonylcarbonsäuren sind seid langem bekannt [A. Schöberl u. A. Wagner in "Methoden der organischen Chemie", Houben-Weyl Bd. IX, S.227 ff. (1955) bzw. M.Quaedvlieg, ebenda, S. 298].

Nach S.Gabriel, Ber.dtsch.chem.Ges.14, 833 (1881) wird z.B. durch Erwärmen von Natriumbenzolsulfinat mit Natriumchloracetat in wäßriger Lösung gemäß folgender Gleichung

$$\text{Aromat-}SO_2Na + ClCH_2CO_2Na \longrightarrow \text{Aromat-}SO_2CH_2CO_2Na + NaCl$$

zunächst das Natriumsalz der Benzolsulfonylessigsäure erhalten. Aus dem Salz läßt sich dann die Sulfonylessigsäure durch Zusatz einer Mineralsäure freisetzen.

Im Gegensatz dazu stehen Untersuchungen von A. Courtin, H. -R. von Tobel und G.Auerbach, Helv.Chim.Acta 63, 1412 (1980), wonach bei 24stündigem Erwärmen von 1 mol 2-Nitrobenzolsulfinsäure, 2 mol Bromessigsäure und 2 mol Natronlauge in wäßriger Losung unter Rückflußbedingungen in 91 %iger Ausbeute nur das Methyl-(2-nitro-phenyl )-sulfon erhalten wird. Offensichtlich wird unter den sauren Reaktionsbedingungen das entstehende Carboxymethyl-arylsulfon sofort zu Methylarylsulfon decarboxyliert.

C.S.Marvel und R.S.Johnson J.org.Chem.13, 822 (1948) bzw. C.S.Marvel und N.A.Meinhardt, J.Amer.chem.Soc.73, 859 (1951) wenden schließlich die "Gabriel'sche Synthesekonzeption" für tensidartige Alkylsulfinate (Octansulfinat bis Octadecansulfinat) an und erhalten durch Carboxymethylierung von molaren Mengen Natriumalkansulfinat mit Natriumchloracetat die Natriumsalze der Alkansulfonylessigsäuren in Ausbeuten zwischen 45 bis 85 %.

Seit kurzem stehen neuartige sulfobetainsubstituierte Sulfinsäuren bzw. deren Salze als potentielle Ausgangsstoffe für die Synthese von neuen sulfobetainsubstituierten ∝-Sulfonylcarbonsäuren als technisch leicht zugängliche Verbindungen zur Verfügung.

Derartige Sulfobetainsulfinate werden durch radikalisch initiierte Sulfocyclosulfinierung von Diallylammoniumsalzen mit Hydrogensulfit nach DD 225 128 A1 bzw. EP 163 319 A3 gewonnen.

Jedoch schlugen nach dem Stand der Technik ausgeführte Versuche fehl, z.B. Natrium-1,1-dimethyl-3-sulfinatomethyl-4-sulfomethyl-pyrrolidinium-betain (Sulfobetainsulfinat) mit Natriumchloracetat zur entsprechenden sulfobetainsubstituierten ∝-Sulfonylessigsäure zu carboxymethylieren. Die erhaltene Reaktionslösung, welche aus einer komplexen Mischung von Ausgangsprodukten, Folgeprodukten und auch dem

Zielprodukt bestand, ließ sich aufgrund des polaren Charakters der Ausgangs- und Reaktionsprodukte nicht zur gewünschten Sulfonylessigsäure aufarbeiten.

Somit existiert kein Verfahren, Sulfobetainsulfinate zu reinen sulfobetainsubstituierten ∝-Sulfonylcarbonsäuren umzusetzen.

Ferner ist auch kein zweistufiges Syntheseverfahren bekannt geworden, welches direkt aus den aus Diallylammoniumsalzen und Hydrogensulfiten in Gegenwart von ∝-Halogencarbonsäuren in situ erzeugten sulfobetainsubstituierten Sulfinaten sulfobetainsubstituierte ∝-Sulfonylcarbonsäuren zugänglich macht.

Gegenstand der Erfindung ist ein einfaches zweistufiges Syntheseverfahren zur Herstellung von sulfobetainsubstituierten ∝-Sulfonylcarbonsäuren aus leicht zugänglichen und technisch verfügbaren Ausgangsstoffen, wie Diallylammoniumsalz, Hydrogensulfit und ∝-Halogencarbonsäuren, das es gestattet, die Verfahrensschritte ohne Bildung von Nebenprodukten bei gleichzeitig hoher Reinheit und Ausbeute des Zielproduktes zu führen.

Die Herstellung der Verbindungen der Formel I erfolgt, indem Diallylammoniumsalze, vorzugsweise Diallylammoniumchloride, der allgemeinen Formel II,

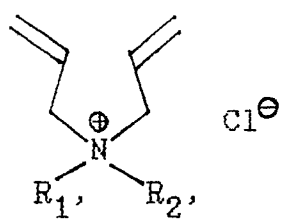

II

in der

$R_1$, und $R_2$, Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellen bzw. Allylreste bedeuten,

mit molaren Mengen einer ∝-Halogencarbonsäure, vorzugsweise ∝-Chlorcarbonsäure, der allgemeinen Formel III,

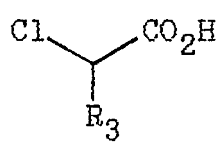

III

in der

$R_3$ die obengenannte Bedeutung hat,

und der doppeltmolaren Menge eines Metallhydrogensulfits, vorzugsweise Natriumhydrogensulfit, in Gegenwart einer katalytischen Menge eines Peroxodisulfats in einem ersten Verfahrensschritt miteinander umgesetzt werden und im sich anschließenden zweiten Verfahrensschritt die erhaltene Reaktionslösung nach Zugabe einer katalytischen Menge eines im Reaktionsmedium löslichen Jodids solange zum Sieden erhitzt wird bis der Sulfinat-Gehalt der reagierenden Lösung auf praktisch Null abgesunken ist.

Die Gesamtreaktion, die radikalisch initiierte Sulfocyclosulfinierung des Diallylammoniumsalzes mit gekoppelter Carboxyalkylierung des in situ gebildeten Sulfobetainsulfinats in zwei Verfahrensstufen, veranschaulicht das folgende Schema:

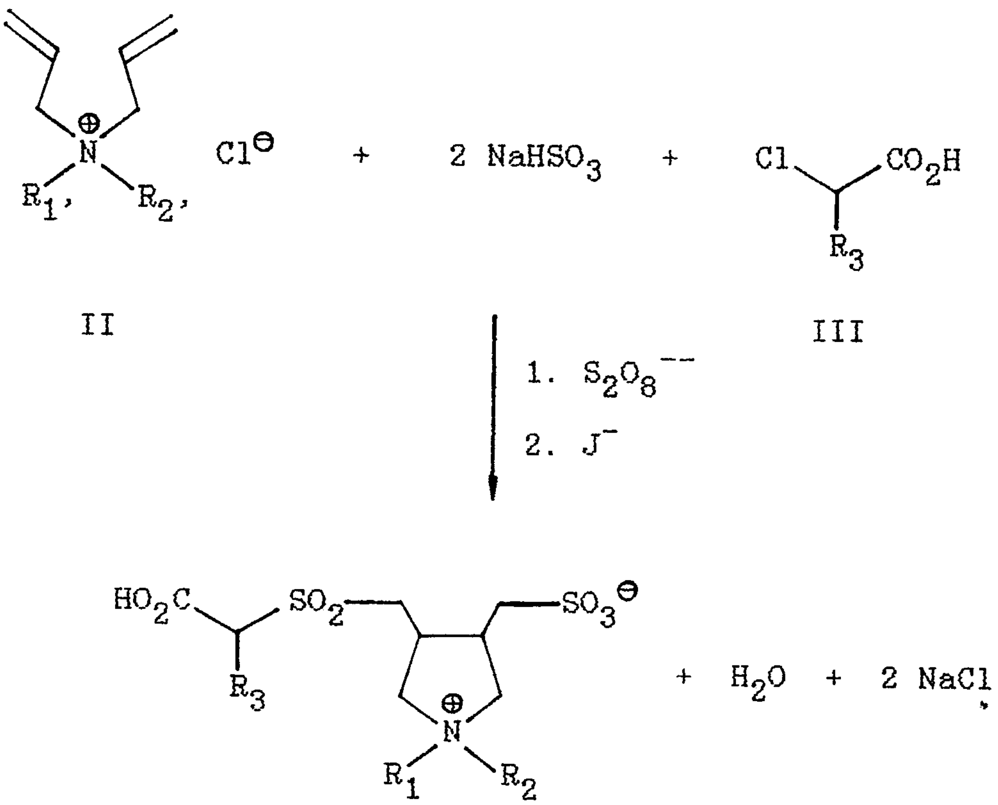


II

III

I

Die Sulfocyclosulfinierung eines Diallyl- bzw.Tetraallylammoniumsalzes verläuft nach DD 225 128 A1 nur dann mit maximaler Ausbeute an Sulfocyclosulfinierungsprodukt (Sulfobetainsulfinat), wenn pH-Bedingungen um 2 eingehalten werden. Das Einstellen auf diesen pH-Wert wird durch Zugabe einer Mineralsäure zur Mischung aus Diallylammoniumsalz und Hydrogensulfit erreicht. Entsprechende pH-Wert-Einstellungen sind bisher nicht mit einer ∝-Halogencarbonsäure vorgenommen worden. Es kann deshalb als ein überraschender Befund angesehen werden, daß beispielsweise der Zusatz von 1 mol Chlorossigsäure zu einer Mischung aus 1 mol Diallylammoniumsalz und 2 mol technischer Natriumhydrogensulfitlösung den optimalen Start-pH-Wert um 2 ergibt; also Bedingungen wie sie der obigen Zielstellung entsprechen. Die Reihenfolge des Zusammengebens der Reaktionsteilnehmer ist für den Reaktionsablauf ohne Bedeutung.

In Abhängigkeit von der Qualität der verwendeten Natriumhydrogensulfitlösung können geringe pH-Wert-Schwankungen auftreten. Sie sind dadurch zu korrigieren, daß entweder eine geringe Menge einer Mineralsäure zugesetzt oder die Reaktion mit einer Teilmenge der Halogencarbonsäure gestartet und die Restmenge der Halogencarbonsäure erst nach der ersten Reaktionsstufe zugesetzt wird.

Bei Einsatz von z.B. tertiären Diallylaminen als Ausgangsprodukte der Umsetzung sollten diese zweckmäßigerweise in der Halogencarbonsäure gelöst und nach Zugabe der Hydrogensulfitlösung der Start-pH-Wert mit einer Mineralsäure eingestellt werden. Da bei tertiären Diallylaminen und einem Start-pH-Wert von 2,5 die Sulfocyclosulfinierung bereits vollständig abläuft, ist weniger als die moläquivalente Menge an Mineralsäure für die Bildung des Diallylammoniumsalzes notwendig.

Zur so vorbereiteten Startlösung, welche eine Temperatur zwischen 20 bis 30 °C haben sollte, wird zur Durchführung des ersten Verfahrensschritts 1 bis 5 Mol-% Peroxodisulfat, bezogen auf das eingesetzte Diallylammoniumsalz, hinzugesetzt, um die Sulfocyclosulfinierungsreaktion auszulösen.

Es hat sich dabei als zweckmäßig erwiesen, das Peroxodisulfat in zwei Portionen hinzuzusetzen, um einen möglichst vollständigen Umsatz zu erreichen, wobei meistens Mengen von 2 mal 2 Mol-%, bezogen auf das eingesetzte Diallylammoniumsalz, völlig ausreichend sind. Als Peroxodisulfate sind Natrium-, Kalium- oder Ammoniumperoxodisulfat geeignet, welche entweder als Lösung oder pulverförmig eingesetzt werden können. Die anfänglich fahlgelbe Startlösung färbt sich nach der Peroxodisulfatzugabe rot bis blutrot, hellt sich aber kurz nach Durchlaufen des Temperaturmaximums deutlich auf.

Zur Einleitung des zweiten Verfahrensschrittes wird zur reagierenden Lösung eine katalytische Menge eines löslichen Jodids, z.B. Natrium-, Kalium- oder Ammoniumjodid, gegebenenfalls die jeweilige ∝-Jodcarbonsäure, z. B. Jodessigsäure, hinzugefügt und solange unter Siedekühlung weiter erwärmt bis in der reagierenden Lösung durch bromatometrische Titration einer Probe kein Sulfinat mehr nachgewiesen

werden kann.

Bei einer Einsatzmenge von 5 bis 50 mmol Natrium-, Kalium- oder Ammoniumjodid/mol Diallylammoniumsalz werden im Verlaufe von ein bis zwei Stunden vollständige Umsätze erreicht. Während Mengen unter 3 mmol der o.g. Jodide keine katalytische Wirkung mehr erkennen lassen, beschleunigen größere Mengen als 7 mmol die Reaktion weiter; verfärben jedoch später die kristallinen Zielprodukte.

Wird die Carboxyalkylierung des Sulfocyclosulfinierungsprodukts ohne jodidzusatz ausgeführt, findet zwar in der Anfangsphase die gewünschte Umsetzung statt, sie verlangsamt sich aber mit fortschreitender Reaktionszeit derart, daß Folgereaktionen nicht ausbleiben und sich dadurch das gewünschte Reaktionsprodukt nicht isolieren läßt, weil auch dieses unter fortwährender thermischer Belastung zersetzt wird.

Überraschend an diesem zweiten Verfahrensschritt ist ferner die Tatsache , daß sich das Sulfocyclosulfinierungsprodukt des Diallylammoniumsalzes bei niedrigem pH-Wert in der Siedehitze carboxyalkylieren läßt, obwohl bekannt ist [W. Jeblick und W. Bunge in "Ullmanns Encyklopädie der technischen Chemie", Bd.22 (1982), S.315], daß unter solchen Bedingungen - niedriger pH-Wert und hohe Temperaturen - Sulfinsäuren bevorzugt zu Thiosulfonsäure-Sester und Sulfonsäuren disproportionieren.

Andererseits wurde es nach dem Stand der Technik für notwendig erachtet, ∝-Sulfonylcarbonsäuren durch Umsetzung der Metallsalze von Sulfinsäuren bzw. ∝-Halogencarbonsäuren zu erhalten, wobei Nebenreaktionen, wie die Decarboxylierung des Zielprodukts, nicht in allen Fällen auszuschließen waren.

Demgegenüber gelingt es in einer besonderen Ausführungsform des Verfahrens den zweiten Syntheseschritt auch separat auszuführen. Dazu kann man entweder zu molaren Mengen isolierter Sulfobetainsulfinsäure und ∝-Halogencarbonsäure ein Moläquivalent einer Base hinzufügen oder das Metallsalz der einen Säure mit der jeweils anderen Säure zur Umsetzung bringen.

Die so erhaltenen Reaktionsprodukte sind mit jenen, welche nach dem zweistufigen Syntheseverfahren gewonnen werden, identisch. Dieser Weg bietet keinen prinzipiellen Verfahrensvorteil, da die dafür notwendigen Ausgangs-Sulfobetainsulfinate zuvor aus den Diallylammoniumsalzen hergestellt werden müssen.

Gegenüber der Arbeitsweise des Standes der Technik werden jedoch in vorteilhafter Weise direkt die freien ∝-Sulfonylcarbonsäuren erhalten, wodurch erst das Neutralisieren und nach erfolgter Umsetzung das Freisetzen der Carbonsäurefunktion vermieden wird. Bei dieser Verfahrensweise werden darüber hinaus Nebenreaktionen verhindert, weshalb sie als wesentlich wirtschaftlicher angesehen werden muß.

Zusammenfassend besteht der Vorteil der Erfindung in einer einfachen Synthese, welche ohne die Isolierung von Zwischenprodukten, die bisher unbekannten sulfobetainsubstituierten ∝-Sulfonylcarbonsäuren der allgemeinen Formel I aus Diallylammoniumsalzen direkt zugänglich macht. Als Ausgangsstoffe werden im technischen Maßstab verfügbare Zwischenprodukte genutzt, die mit einfachen Mitteln in kurzen Reaktionszeiten zur Umsetzung gebracht werden können.

Die Erfindung soll durch die nachfolgenden Beispiele näher erläutert werden.

Beispiel 1

1, 1-Dimethyl-3-carboxymethylsulfonylmethyl-4-sulfomethyl-pyrrolidinium-betain

($R_1$ = $R_2$ = $CH_3$; $R_3$ = H in der allgemeinen Formel I)

a) In einem 1,5 1 Sulfierkolben, der mit einem Rührer, Thermometer, Rückflußkühler sowie einer Heizquelle ausgerüstet ist, werden 307,4 g (1 mol) 52,6 %ige wäßrige Dimethyldiallylammoniumchloridlösung, 118,1 g (1 mol) 80 %ige wäßrige Chloressigsäure sowie 539 g (2,02 mol) 39 %ige technische Natriumhydrogensulfitlösung vorgelegt und miteinander unter Rühren vermischt. Man erhält eine fahlgelbe, homogene Lösung von 21 °C und einen Start-pH-Wert von 2,02. Zur Initiierung der Umsetzung fügt man zunächst 2 Mol-% (4,56 g) Ammoniumperoxodisulfat und eine Minute später - die Reaktionstemperatur ist inzwischen auf 60 °C gestiegen und die Reaktionslösung hat sich blutrot gefärbt - nochmals 2 Mol-% Ammoniumperoxodisulfat hinzu. Bereits nach einer weiteren Minute wird das Reaktionstemperaturmaximum von 78 °C erreicht. Man fügt zur Reaktionslösung 1,05 g (7 mmol) Natriumjodid hinzu und erwärmt für 90 Minuten zum Sieden auf 110 °C. Schon während des Hochheizens entfärbt sich die rote Reaktionslösung und ist beim Erreichen der Siedetemperatur fahlgelb. Die Rotfärbung der Lösung wird durch Eisen-(III)-1,1-dimethyl-3-sulfinatomethyl-4-sulfomethyl-pyrrolidinium-betain hervorgerufen, welches aus dem Reaktionszwischenprodukt 1,1-Dimethyl-3-sulfinatomethyl-4-sulfomethyl-pyrrolidinium-betain sowie den in der verwendeten technischen Hydrogensulfitlösung vorhandenen Eisensalzspuren entsteht. Zu diesem Zeitpunkt ist die Umsetzung bereits quantitativ beendet, d.h. das Reaktionszwischenprodukt 1,1-Dimethyl-3-sulfinatomethyl-4-sulfomethyl-pyrrolidinium-betain kann durch bromatometrische Titration einer Probe der Reaktionslösung nicht mehr nachgewiesen werden. (Wird die Umsetzung jedoch ohne Zusatz von Jodid durchgeführt, so beträgt der Umsatz nach 90 Minuten 86 % und nach 360 Minuten 94 %, parallel dazu erfogt eine beträchtliche Decarboxylierung des Zielprodukts.) Um die spätere Ausbeute an kristallinem Zielprodukt zu erhöhen, kann bereits während der Siedephase zwecks Aufkonzentrierung

Wasser von der Reaktionslösung abdestilliert werden. Zur vollständigen Kristallisation läßt man abkühlen und über Nacht stehen. Zur Aufarbeitung saugt man den Kristallbrei scharf von der Mutterlauge ab, wäscht die Kristalle mit wenig Wasser und zweimal mit Alkohol und erhält nach dem Trocknen an der Luft 250 g eines weißen, lockeren Kristallpulvers, welches zur Feinreinigung noch aus Wasser umkristallisiert werden kann. Man erhält ein sehr reines 1,1-Dimethyl-3-carboxymethylsulfonylmethyl-4-sulfomethyl-pyrrolidinium-betain-dihydrat (F: ab 290 °C unter Zersetzung). $^{13}C$-NMR-Spektrum des Natriumsalzes ($H_2O$; externer Standard Tetramethylsilan - TMS; $\delta$ = 0,0 ppm):

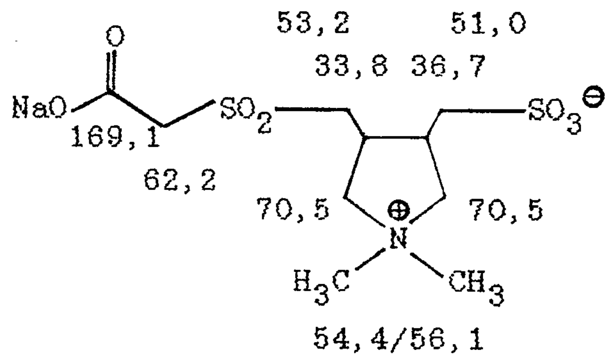

Die Zahlenangaben an den Atomsymbolen entsprechen den chemischen Verschiebungen für die cis-Konfiguration (3,4-Position) in ppm. Die $N-CH_3$-Gruppen sind nicht äquivalent und wie bei den $N-CH_2$-Gruppen erfolgt noch zusätzlich eine Signalaufspaltung durch das $^{14}N$-Quadrupolmoment. Demgegenüber zeigt ein in $D_2O$ aufgenommenes $^{13}C$-NMR-Spektrum, daß die Methylengruppe zwischen der Carboxylat- und Sulfongruppe einen ausgeprägten CH-aciden Charakter besitzt, was durch eine starke Intensitätsverringerung des Signals bei 62,2 ppm infolge H/D-Austauschs erkennbar ist.

b) 30,7 g (0,1 mol) 1,1-Dimethyl-3-sulfinsäuremethyl-4-sulfomethyl-pyrrolidinium-betain-dihydrat (hergestellt aus Dimethyldiallylammoniumchlorid und Natruimhydrogensulfit; vgl. DD 225 128 A1, Beispiel 13), 12,1 g (0,1 mol) 33%ige Natronlauge, 11,8 g (0,1 mol) 80 %ige wäßrige Chloressigsäure, 0,15 g (1 mmol) Natriumjodid und 20 g Wasser werden miteinander vermischt und 30 Minuten zum Sieden erwärmt. Aus der anfangs klaren, farblosen Lösung fallen schon nach kurzer Reaktionszeit Kristalle des Zielprodukts aus. Nach Abkühlung der Reaktionslösung kann das Zielprodukt in über 90 %iger Ausbeute als Dihydrat isoliert werden. Das $^{13}C$-NMR-Spektrum des Natriumsalzes des erhaltenen kristallinen Produkts ist mit dem nach Beispiel 1a hergestellten völlig identisch.

Beispiel 2

1,1-Dimethyl-3-(1'-carboxyethylsulfonylmethyl)-4-sulfomethyl-pyrrolidinium-betain
($R_1$ = $R_2$ = $R_3$ = $CH_3$ in der allgemeinen Formel I)

Man verfährt nach Beispiel 1a und setzt eine Mischung aus 61,5 g (0,2 mol) 52,6 %ige wäßrige Dimethyldiallylammoniumchloridlösung, 107,8 g (0,404 mol) 39 %ige technische Natriumhydrogensulfitlösung und 21,7 g (0,2 mol) 2-Chlorpropionsäure - die 2-Chlorpropionsäure ist bei der Mischungstemperatur der Reaktionspartner von 28 °C nicht vollständig gelöst; der pH-Wert der Mischung stellt sich auf 2,3 ein - mit zwei Portionen von je 0,91 g (2 Mol-%) Ammoniumperoxodisulfat um. Nach Erreichen des Reaktionstemperaturmaximums von 79 °C in 90 Sekunden wird zur inzwischen zitronengelbgefärbten Lösung 0,2 g (1,3 mmol) Natriumjodid hinzugefügt und 2 Stunden zum Sieden erhitzt. Man bewahrt die abgekühlte Reaktionslösung für einige Zeit im Kühlschrank auf und isoliert das Zielprodukt in etwa 60 %iger Ausbeute in wohlausgebildeten, farblosen Kristallen. Zur Feinreinigung kann noch aus Wasser umkristallisiert werden (F: 215 bis 218 °C). Durch Aufarbeitung der Mutterlauge kann noch eine zweite kristalline Fraktion des Zielprodukts erhalten werden. Wegen der Schwerlöslichkeit der freien Carbonsäure in Wasser bei Raumtemperatur wird eine Probe durch Umsetzung mit Calciumcarbonat in das leichter lösliche Calciumsalz überführt (F: ab 270 °C unter Zersetzung).

$^{13}C$-NMR-Spektrum des Calciumsalzes
($D_2O$; externer Standard TMS; $\delta$ = 0,0 ppm):

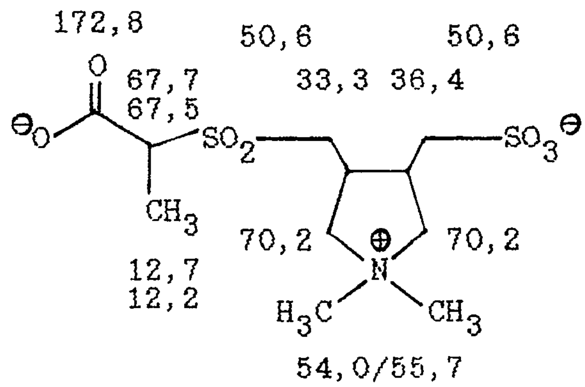

Die N-$CH_3$-Gruppen sind nicht äquivalent und wie bei den $CH_2$-Gruppen erfolgt noch zusätzliche Signalaufspaltung durch das $^{14}N$-Quadrupolmoment. Die Signalverdoppelungen bei 67,5/67,7 ppm bzw. 12,2/12,7 ppm ist auf die verschiedenen Asymmetriezentren des Moleküls zurückzuführen.

Beispiel 3
1,1-Dimethyl-3-(1'-carboxypropylsulfonylmethyl)-4-sulfomethyl-pyrrolidinium-betain
($R_1$ = $R_2$ = $CH_3$; R3 = $C_2H_5$ in der allgemeinen Formel I)

Man verfährt nach den Beispielen 1 und 2 und erhält das Zielprodukt in über 90 %iger Ausbeute in Form von farblosen Kristallen (F.: 198 - 200 °C; F. des Calciumsalzes: 225 - 227 °C).
$^{13}C$-NMR-Spektrum des Calciumsalzes ($D_2O$; externer Standard TMS; $\delta$ = 0,0 ppm):

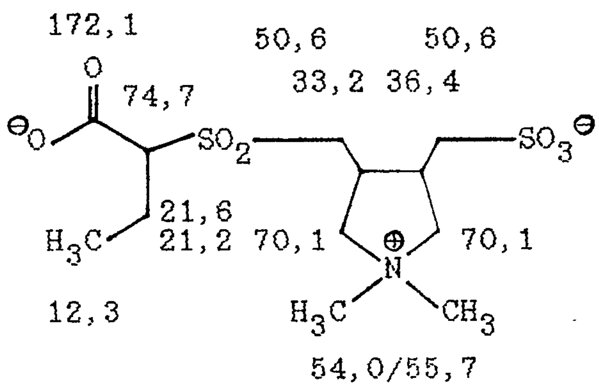

Die N-$CH_3$-Gruppen sind nicht äquivalent und wie bei den N-$CH_2$-Gruppen erfolgt noch zusätzlich Signalaufspaltung durch das $^{14}N$-Quadrupolmoment. Die Signalverdoppelung bei 21,2/21,6 ppm ist auf die verschiedenen Asymmetriezentren des Moleküls zurückzuführen.

Beispiel 4
1,1-Dimethyl-3-(1'-carboxypentylsulfonylmethyl)-4-sulfomethyl-pyrrolidinium-betain
($R_1$ = $R_2$ = $CH_3$; $R_3$ = $C_4H_9$ in der allgemeinen Formel I)

Man verfährt nach Beispiel 1b und setzt eine Mischung aus 15,35 g (50 mmol) 1,1-Dimethyl-3-sulfinsäuremethyl-4-sulfomethyl-pyrrolidinium-betain, 9,75 g (50 mmol) 2-Bromhexansäure, 2,5 g (25 mmol) Calciumcarbonat, 0,3 g (2 mmol) Natriumjodid und 10 g Wasser unter Erwärmen - anfangs unter Kohlendioxidentwicklung - miteinander um; bis der Sulfinatgehalt der Lösung auf praktisch Null abgefallen ist. Danach fügt man noch 2,5 g (25 mmol) Calciumcarbonat hinzu und überläßt das sich bildende Calciumsalz der Kristallisation. Durch Nachwaschen mit Alkohol erhält man ein vom Natriumjodid befreites, farbloses Calciumsalz des Zielproduktes in über 90 %iger Ausbeute, welches zur Feinreinigung aus Wasser umkristallisiert werden kann.
F.: ab 260 °C unter Zersetzung.
$^{13}C$-NMR-Spektrum des Calciumsalzes ($D_2O$; externer Standard TMS;

δ = 0,0 ppm):

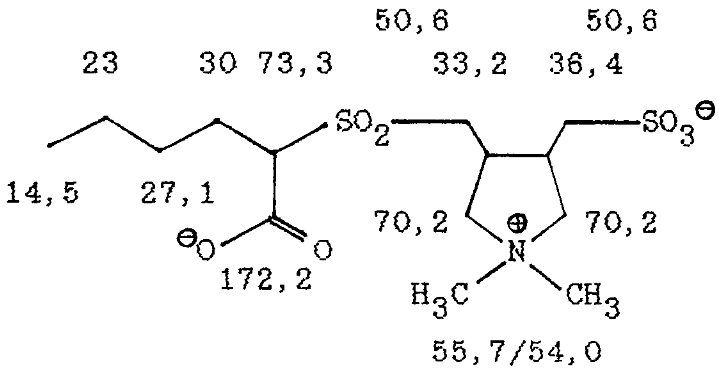

Die N-$CH_3$-Gruppen sind nicht äquivalent (cis-Verbindung - in Bezug auf die Substituenten in der 3- bzw. 4-Position) und wie bei den N-$CH_2$-Gruppen erfolgt noch zusätzlich Signalaufspaltung durch das $^{14}N$-Quadrupolmoment.

Beispiel 5

Natruim-1,1,-(2'/3'-carboxymethylsulfonylmethyl-3'/2'-sulfomethyl) tetramethylen-3-carboxymethylsulfonylmethyl-4-sulfomethyl-pyrrolidinium-betain-Isomerengemisch aus Tetraallylammoniumbromid

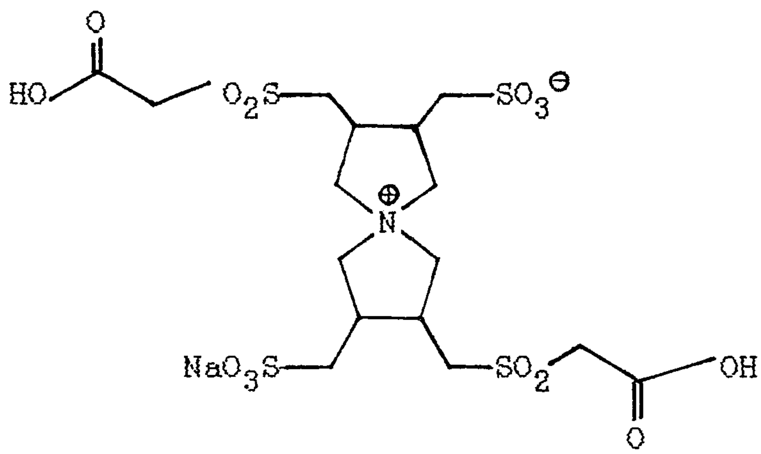

Man verfährt nach Beispiel 1a und setzt eine Mischung aus 25,8 g (0,1 mol) Tetraallylammoniumbromid gelöst in 25 g Wasser, 107,8 g (0,404 mol) 39 %ige technische Natriumhydrogensulfitlösung und 23,6 g (0,2 mol) 80 %ige Chloressigsäure - pH-Wert der Mischung 2,2 - mit zwei Portionen von je 0,46 g (2 Mol-%) Ammoniumperoxodisulfat um. Dabei steigt die Reaktionstemperatur von 25 auf 64 °C an. Nach Zusatz von 0,15 g (1 mmol) Natriumjodid und zweistündigen Erhitzen zum Sieden kann das Zwischenprodukt bromatometrisch nicht mehr nachgewiesen werden. Man engt die Raktionslösung ein, behandelt den Rückstand mit Methanol und erhält nach dem Trocknen an der Luft eine farblose kristalline Substanz (F.: 210 °C).

Beispiel 6

1-Dodecyl-3-carboxymethylsulfonylmethyl-4-sulfomethyl-pyrrolidinium-betain
($R_1$ = $R_3$ = H; $R_2$ = $C_{12}H_{25}$ in der allgemeinen Formel I)

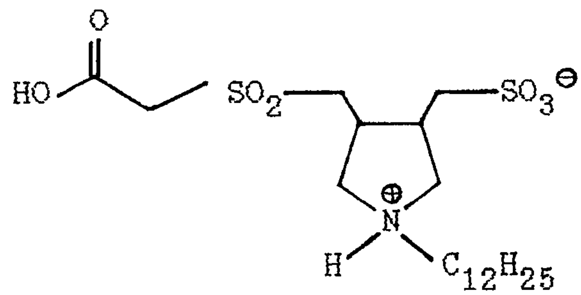

Man verfährt nach Beispiel 1 a und setzt eine Mischung von pH 2 aus 26,55 g (0,1 mol) Diallyldodecylamin, 11,8 g (0,1 mol) 80 %ige wäßrige Chloressigsäure, 53,9 g (0,202 mol) 39 %ige technische Natriumhydrogensulfitlösung und 7 g 37 %iger Salzsäure mit 2 Portionen von je 0,46 (2 Mol-%) Ammoniumperoxodisulfat um. Dabei steigt die Reaktionstemperatur von 24 auf 62 °C an. Man erhält eine viskose, klar gelbe Lösung, die nach Zusatz von 0,15 g (1 mmol) Natriumjodid sowie zweistündigem Erwärmen auf 106 °C quantitativ zum Zielprodukt umgesetzt ist. Nach dem Abkühlen trennt sich die Reaktionslösung in zwei Phasen, die obere enthält das Zielprodukt als viskose Lösung.

Beispiel 7

1-Dodecyl-1-methyl-3-carboxymethylsulfonylmethyl-4-sulfomethyl-pyrrolidinium-betain
($R_1$ = $CH_3$; $R_2$ = $C_{12}H_{25}$; R3 = H in der allgemeinen Formel I)

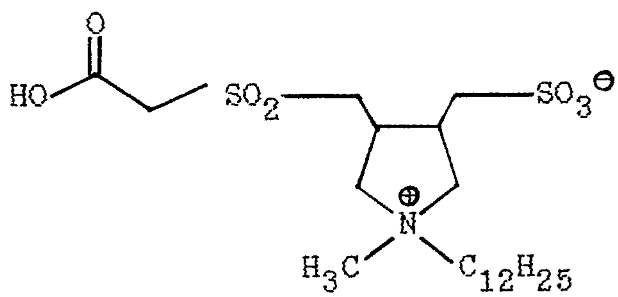

Man verfährt nach den Beispielen 1a und 6 und setzt eine Mischung von pH 2 aus 36,04 g (0,1 mol) 80 %ige Dodecylmethyldiallylammoniumbromid, 11,8 g (0,1 mol) 80 %ige Chloressigsäure sowie 53,9 g (0,202 mol) 39 %ige Natriumhydrogensulfitlösung mit zwei Portionen von je 0,46 g (2 Mol-%) Ammoniumperoxodisulfat um. Anschließend fügt man 0,15 g (1 mmol) Natriumjodid hinzu und erwärmt für zwei Stunden zum Sieden unter Rückflußbedingungen. Man erhält eine bernsteinfarbene, homogene Lösung des Zielprodukts. Aus dieser Lösung kann durch Extraktion mit n-Butanol, anschließender Abdestillation des n-Butanols, lösen des Rückstands unter leichtem Erwärmen in Ethanol sowie kristallisieren lassen, ein weißes Kristallpulver, welches sich ab 156 °C zu zersetzen beginnt, in über 90 %iger Ausbeute isoliert werden. Die pulverförmige α-Sulfonylessigsäure ist in Wasser (20 °C) ziemlich schwer löslich, jedoch unter Schütteln mit verdünnter Natronlauge leicht löslich sowie stark schäumend.

**Ansprüche**

1. Sulfobetainsubstituierte α-Sulfonylcarbonsäuren aus Diallylammoniumsalzen der allgemeinen Formel I,

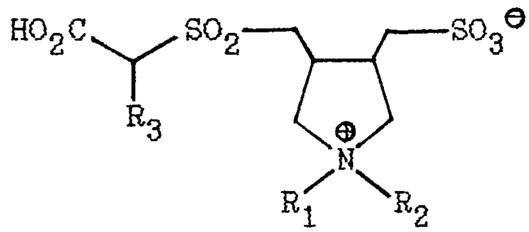

I

worin
$R_1$ und $R_2$ Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellen, oder einen -(2'/3'-Carboxymethylsulfonylmethyl-3'/2'-sulfomethyl)tetramethylen-Rest bedeuten,
und
$R_3$ Wasserstoff, einen geradkettigen oder verzweigten Alkylrest mit 1 bis 22 Kohlenstoffatomen oder einen Alkylrest, welcher am Kettenanfang die Gruppe $-CH_2-COH-$ enthalten kann, darstellt.

**2.** Verbindung nach Anspruch 1, worin $R_1$ und $R_2$ $-CH_3$ und $R_3$ Wasserstoff bedeuten.

**3.** Verbindung nach Anspruch 1, worin $R_1$, $R_2$ und $R_3$ $-CH_3$ bedeuten.

**4.** Verbindung nach Anspruch 1, worin $R_1$ und $R_2$ $-CH_3$ und $R_3$ $-C_2H_5$ bedeuten.

**5.** Verbindung nach Anspruch 1 , worin $R_1$ und $R_2$ $-CH_3$ und $R_3$ $-C_4H_9$ bedeuten.

**6.** Verbindung nach Anspruch 1, worin $R_1$ und $R_3$ Wasserstoff und $R_2$ $-C_{12}H_{25}$ bedeuten.

**7.** Verbindung nach Anspruch 1, worin $R_1$ $-CH_3$, $R_2$ $-C_{12}H_{25}$ und $R_3$ Wasserstoff bedeuten.

**8.** Verfahren zur Herstellung von sulfobetainsubstituierten $\alpha$-Sulfonylcarbonsäuren aus Diallylammoniumsalzen der allgemeinen Formel I nach Anspruch 1 bis 7, wobei Diallylammoniumsalze, vorzugsweise Diallylammoniumchloride, der allgemeinen Formel II

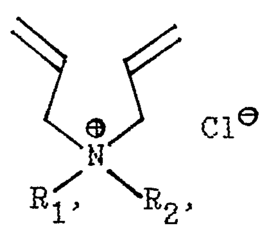

II

in der
$R_{1'}$ und $R_{2'}$ Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellen bzw. Allylreste bedeuten,
mit molaren Mengen einer $\alpha$-Halogencarbonsäure, vorzugsweise $\alpha$-Chlorcarbonsäure, der allgemeinen Formel III,

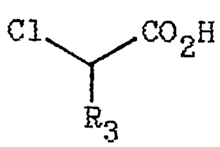

III

in der
$R_3$ die obengenannte Bedeutung hat,
und der doppeltmolaren Menge eines Metallhydrogensulfits, vorzugsweise Natriumhydrogensulfit, in

Gegenwart einer katalytischen Menge eines Peroxodisulfats im ersten Verfahrensschritt miteinander umgesetzt und im zweiten Verfahrensschritt die erhaltene Reaktionslösung nach Zugabe einer katalytischen Jodidmenge solange zum Sieden erhitzt wird bis der Sulfinat-Gehalt der reagierenden Lösung auf Null abgesunken ist.

**9.** Verfahren nach Anspruch 8, gekennzeichnet dadurch, daß im ersten Verfahrensschritt 1 bis 5 Mol-% Peroxodisulfat, bezogen auf das eingesetzte Diallylammoniumsalz, als Katalysator zugesetzt werden und im zweiten Terfahrensschritt 5 bis 50 mmol Natruim-, Kalium- oder Ammoniumjodid/mol Diallylammoniumsalz als Katalysator zugesetzt werden.

**10.** Verfahren nach Anspruch 8 und 9, gekennzeichnet dadurch, daß das Peroxodisulfat in zwei Portionen von jeweils 2 Mol-%, bezogen auf das eingesetzte Diallylammoniumsalz, zugesetzt wird.

Europäisches Patentamt

# EUROPÄISCHER RECHERCHENBERICHT

Nummer der Anmeldung

**EP 90 25 0322**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | GB-A-2 080 293 (AKADEMIE D. WISSENSCHAFTEN) * Insgesamt * | 1-10 | C 07 D 207/08 |
| A | US-A-4 528 383 (SCHMITT) * Insgesamt * | 1-10 | |
| D,A | EP-A-0 163 319 (AKADEMIE D. WISSENSCHAFTEN) * Insgesamt * | 1-10 | |

RECHERCHIERTE SACHGEBIETE (Int. Cl.5)

C 07 D 207/00

Der vorliegende Recherchenbericht wurde für alle Patentansprüche erstellt

| Recherchenort | Abschlußdatum der Recherche | Prüfer |
|---|---|---|
| Den Haag | 15 März 91 | KISSLER B.E. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument